# EUROPEAN PATENT APPLICATION

(11) **EP 1 221 613 A2**
(43) Date of publication of application: **10.07.2002**
(21) Application number: 02250010.2
(22) Date of filing: 02.01.2002
(51) Int. Cl.: G01N 33/08

(54) **Method and apparatus for inspecting the surface condition of egg shells**

(30) Priority: 05.01.2001 JP 2001000824
(71) Applicant: Nabel Co., Ltd., Nagaokakyo-City, Kyoto 617-0836 (JP)
(72) Inventor: Nambu, Kunio, c/o Nabel Co. Ltd., Nagaokakyo-City, Kyoto 617-0836 (JP)
(74) Representative: Senior, Alan Murray

(57) **Abstract**

An apparatus for detecting the surface condition of egg shells includes: a radiating device for radiating to eggs a plurality of electromagnetic waves whose wavelengths are mutually different and which have substantially line spectra; and a detecting device for detecting the surface condition of the egg shells on the basis of intensities of reflected electromagnetic waves reflected from the eggs due to the radiation by the radiating device.

## Description

### BACKGROUND OF THE INVENTION

### Field of the Invention:

The present invention relates to a method of detecting the surface condition of egg shells such as the degree of stain on or dirt adhering to surfaces of egg shells and their types, as well as an apparatus for detecting the surface condition of egg shells using this method.

### Description of the Related Art:

Generally, in cases where eggs are to be shipped to the market, the eggs collected from a producing district are cleaned (washed), after cleaning they are sorted according to ranges of weight, and after sorting they are packaged (packed or tray packed) in units of fixed numbers or fixed weight. At present, these washing, sorting, and packaging operations are automated practically completely and are effected at high speed.

If cracked eggs are present among the eggs collected from the producing district, and yolks have flowed out on the egg shell surfaces, the washing, sorting, and packaging processes are contaminated. Hence, there are possibilities that such processes must be stopped temporarily, and the market value of products with cracked eggs packed or tray packed therein declines appreciably, and they are unable to be shipped as products.

Accordingly, there is a need to specify cracked eggs among the eggs collected from the producing district and to remove them. However, according to a method in which mechanical shocks are imparted to the eggs by striking them with a striking rod or the like, and the cracked eggs are specified on the basis of vibrational noise or the like occurring due to these shocks, there is the risk that cracks can be caused to normal eggs during the imparting of the shocks. Meanwhile, according to a method in which eggs collected from a producing district are merely imaged by an imaging device, and cracked eggs are specified by the adherence of the yolk on egg shell surfaces by analyzing the picked-up image, in cases where other dirt such as chicken droppings adheres to the egg shell surfaces, it is difficult to discriminate cracked eggs from such dirty eggs . Hence, there is a possibility that eggs which are uncracked may be specified as cracked eggs.

In addition, in cases where eggs with other large dirt such as chicken droppings adhering to egg shell surfaces are packed or tray packed, there is the possibility that the market value of such products declines appreciably, and they are unable to be shipped as products in the same way as the case in which cracked eggs are mixed in. However, with the aforementioned method in which shocks are imparted to the eggs, the eggs with large dirt such as chicken droppings adhering to egg shell surfaces are difficult to specify among the eggs collected from the producing district. And the discrimination of the dirty eggs from cracked eggs is difficult by the above-described image analysis. As a result, there are cases where those eggs which, unlike cracked eggs, can be used as products if the other dirt such as chicken droppings is removed from the egg shell surfaces are bound to be handled in the same way as the cracked eggs.

### SUMMARY OF THE INVENTION

The present invention has been devised in view of the above-described aspects, and its object is to provide a method of detecting the surface condition of an egg shell which makes it possible to detect accurately and with extremely high reliability the surface condition of the egg shell such as stain on or dirt adhering to the egg shell surface without imparting shocks to the egg, as well as an apparatus for detecting the surface condition of an egg shell using this method.

To this end, in accordance with an aspect of the invention, there is provided a method of detecting the surface condition of an egg shell, comprising the steps of: radiating to an egg a plurality of electromagnetic waves whose wavelengths are mutually different and each of which has substantially line spectrum; and detecting the surface condition of the egg shell on the basis of an intensity of a reflected electromagnetic wave from the egg due to the radiation.

According to the method of the invention, since the surface condition of the egg shell is detected on the basis of the intensity of the reflected electromagnetic wave after radiating the electromagnetic wave to the egg, no mechanical shocks are imparted to the egg, and no new cracks are caused in the egg shell in performing the detection. Moreover, since the surface condition of the egg shell is detected on the basis of the intensity of the reflected electromagnetic waves based on the electromagnetic waves having different wavelengths, it is possible to discriminate and detect accurately and with extremely high reliability the surface condition of the egg shell such as different stain or dirt adhering to the egg shell surface.

As for the electromagnetic wave in accordance with the invention, it suffices if it has substantially line spectrum, and therefore the line spectrum may have slight range. In the invention, the plurality of electromagnetic waves each having such substantially line spectrum may be radiated to the egg in a time-sharing manner, simultaneously, or independently of each other. Here, as the electromagnetic waves to be radiated, it is possible to cite rays of light, preferably rays of visible light, and it is possible to cite as a preferred example rays of light selected from rays of red light, blue light, and green light in order to detect the surface condition of the egg shell concerning a plurality of kinds of stain or dirt having mutually different saturations. In order to detect the surface condition of the egg shell by discriminating the stain or dirt due to the yoke, chicken droppings, and other matter on the egg shell surface, it is possible to cite the rays of red light and blue light as a particularly preferred example. However, the invention is not limited to the same, and infrared rays or far infrared rays may be used as the electromagnetic wave to be radiated in order to detect the surface condition of the egg shell by discriminating the stain or dirt having differences among the egg shell concerning, for example, the coefficient of thermal absorption, such as the stain or dirt due to the leakage of the white.

In the invention, the surface condition of the egg shell to be detected may include at least one of the presence or absence of the stain or dirt on the egg shell surface, the type of stain or dirt on the egg shell surface, and the degree of stain or dirt on the egg shell surface, but may include other surface conditions of the egg shell, such as the breakage of the egg shell, the presence or absence of spots on the egg shell surface and the like.

In the method of the invention, an arrangement may be provided such that electromagnetic wave is radiated to a stationary egg, and the surface condition of the egg shell is detected on the basis of the intensity of the reflected electromagnetic wave from the stationary egg. Preferably, however, the egg may be rotated, and the electromagnetic wave may be radiated to the egg which is rotated in correspondence with the rotation, or the egg may be conveyed in conjunction with the rotation or without being rotated, and the electromagnetic wave may be radiated to the egg which is conveyed in correspondence with the conveyance.

By rotating the egg, it is possible to easily detect the conditions of the overall surface of the egg shell, and by conveying the egg, the method of the invention can be easily incorporated into the washing, sorting, and packing operations.

In the method of the invention, preferably, image data on the egg may be generated on the basis of the intensity of the reflected electromagnetic wave, and the surface condition of the egg shell may be detected from the image data. Here, the image data on the egg may includes light-and-dark image data. In the case where such light-and-dark image data is used, it suffices if the surface condition of the egg shell is detected on the basis of one piece of light-and-dark image data on the egg based on the intensity of one reflected electromagnetic wave and another piece of light-and-dark image data on the egg based on the intensity of another reflected electromagnetic wave having a wavelength different from that of the one reflected electromagnetic wave. In this case, it suffices if the surface condition of the egg shell is detected on the basis of light and dark in the one piece of light-and-dark image data and light and dark in the other piece of light-and-dark image data, and if the surface condition of the egg shell is detected on the basis of at least one of a proportion of a light area and a proportion of a dark area in at least one of the one piece of light-and-dark image data and the other piece of light-and-dark image data.

The apparatus for detecting the surface condition of an egg shell in accordance with the invention implements the above-described method, and basically comprises: radiating means for radiating to egg a plurality of electromagnetic waves whose wavelengths are mutually different and each of which has substantially line spectrum; and detecting means for detecting the surface condition of the egg shell on the basis of an intensity of a reflected electromagnetic wave reflected from the egg due to the radiation by the radiating means.

In such an apparatus for detecting the surface condition of an egg shell of the present invention, the radiating means may radiate the plurality of electromagnetic waves whose wavelengths are mutually different to the egg in a time-sharing manner, radiate the plurality of electromagnetic waves whose wavelengths are mutually different to the egg simultaneously, or radiate the plurality of electromagnetic waves whose wavelengths are mutually different to the egg independently of each other. In addition, the radiating means may radiate rays of light as the electromagnetic wave, radiate rays of visible light as the rays of light, radiate, as the rays of visible light, rays selected from those of red light, blue light, and green light, or radiate rays of red light and blue light as the rays of visible light.

The radiating means in accordance with the invention may preferably have a plurality of light emitting elements disposed in such a manner as to radiate the emitted rays of light to the egg. The plurality of light emitting elements preferably include a red-light emitting element for emitting red light and a blue-light emitting element for emitting blue light. In the case where the plurality of red-light emitting elements and the plurality of blue-light emitting elements are used, the radiating means may be formed by being provided with a substrate on which these light emitting elements are alternately arranged and mounted.

As the light emitting element, it is possible to cite a light emitting diode as a preferred example, but a white lamp, a plasma element, and other light emitting elements may be used. In the case where red light and blue light are emitted by white lamps, it is preferable to use those with filters. By using the plurality of light emitting elements for radiating the respective rays of light, it is possible to effect the radiation of light having planar and large energy, so that the surface condition of the egg shell can be detected more accurately and with extremely high reliability.

In a preferred example, as the surface condition of the egg shell, the detecting means in accordance with the invention is adapted to detect at least one of the presence or absence of the stain or dirt on the egg shell surface, the type of stain or dirt on the egg shell surface, and the degree of stain or dirt on the egg shell surface, but may be adapted to detect other surface conditions of the egg shell.

The apparatus for detecting the surface condition of an egg shell of the present invention may further comprise at least one of rotating means for rotating the egg and conveying means for conveying the egg. In a preferred example, a rotating/conveying means for conveying the egg while rotating the egg is provided. Here, the radiating means may radiate the electromagnetic wave in correspondence with the rotation of the egg by the rotating means or the conveyance of the egg by the conveying means. In this case, the apparatus for detecting the surface condition of an egg shell in accordance with the invention may further comprise: conveyance detecting means for detecting the conveyance of the egg by the conveying means, wherein the radiating means radiates the electromagnetic waves in response to a detection signal from the conveyance detecting means, and the detecting means detects the surface condition of the egg shell in response to the detection signal from the conveyance detecting means.

It should be noted that, instead of radiating the electromagnetic wave in correspondence with the rotation or conveyance of the egg, the rotating means and the conveying means may be so arranged as to rotate the egg and convey the egg in correspondence with the radiation of the electromagnetic wave. Furthermore, the radiation of the electromagnetic wave by the radiating means, the rotation of the egg by the rotating means, and the conveyance of the egg by the conveying means may be effected in parallel in synchronism with each other.

In a preferred example, the conveying means in accordance with the invention has a plurality of freely rolling members arranged in a conveying direction of the egg and a direction perpendicular to the conveying direction and traveling means for causing the freely rolling members to travel, the egg being held between the freely rolling members which are adjacent to each other in the conveying direction.

In a preferred example, the detecting means in accordance with the invention has a sensing and generating device for sensing the intensity of the reflected electromagnetic waves from the egg and generating image data on the egg, and analyzing means for analyzing the surface condition of the egg shell on the basis of the image data from the sensing and generating device. The sensing and generating device may generate light-and-dark image data as the image data on the egg. In a case where the light-and-dark image data is generated, the sensing and generating device may generate one piece of light-and-dark image data on the egg based on the intensity of one reflected electromagnetic wave and another piece of light-and-dark image data on the egg based on the intensity of another reflected electromagnetic wave having a wavelength different from that of the one reflected electromagnetic wave, and the analyzing means may analyze the surface condition of the egg shell on the basis of the two pieces of light-and-dark image data. In addition, the analyzing means may determine the surface condition of the egg shell on the basis of light and dark in the one piece of light-and-dark image data and light and dark in the other piece of light-and-dark image data. Alternatively, or in combination therewith, the analyzing means may determine the surface condition of the egg shell on the basis of at least one of a proportion of a light area and a proportion of a dark area in at least one of the one piece of light-and-dark image data and the other piece of light-and-dark image data.

As a preferred example of the sensing/generating device, it is possible to cite an imaging device, and such an imaging device is not limited to a color camera and may be a monochrome camera (black-and-white camera). Further, an arrangement may be provided such that a plurality of cameras are disposed so as to sense electromagnetic waves having mutually different wavelengths, preferably rays of visible light having mutually different wavelengths. As a preferred example of the analyzing means, it is possible to cite a computer which is controlled by a program and has a storage unit, an arithmetic unit, and the like, but the invention is not limited to such a computer, and a specifically designed analyzer may be used.

In accordance with the invention, it is possible to provide a method of detecting the surface condition of an egg shell which makes it possible to detect accurately and with extremely high reliability the surface condition of an egg shell such as stain on or dirt adhering to the egg shell surface without imparting shocks to the egg, as well as an apparatus for detecting the surface condition of an egg shell using this method.

Hereafter, a detailed description will be given of the embodiment of the invention with reference to the accompanying drawings. It should be noted that the invention is not limited to the embodiment.

### BRIEF DESCRIPTION OF THE DRAWINGS

Fig. 1 is an explanatory side view of an embodiment of the invention;
Fig. 2 is an explanatory side cross-sectional view of the embodiment shown in Fig. 1;
Fig. 3 is an explanatory plan view, partially cutaway, of the embodiment shown in Fig. 1;
Fig. 4 is an explanatory diagram of mainly a radiating means of the embodiment shown in Fig. 1;
Fig. 5 is a block diagram concerning control in the embodiment shown in Fig. 1; and
Fig. 6 is an explanatory diagram of another example of the radiating means in the embodiment of the invention.

### DESCRIPTION OF THE PREFERRED EMBODIMENT

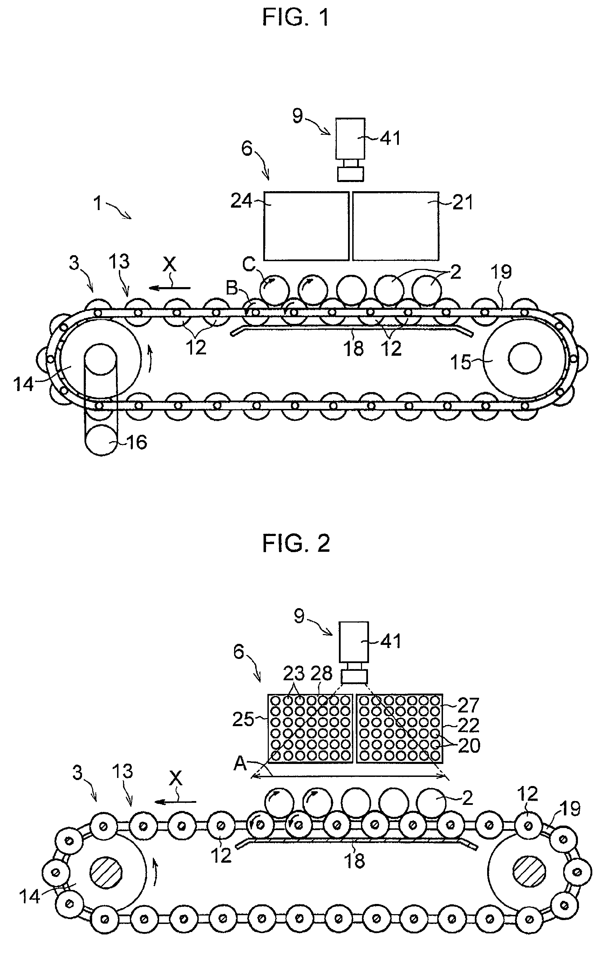

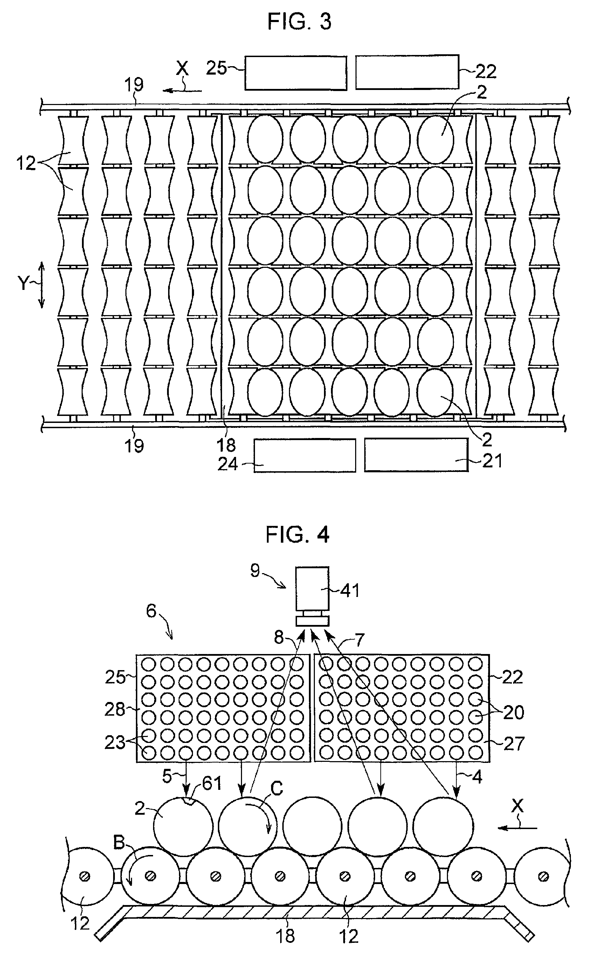

In Figs. 1 to 5, an apparatus 1 for detecting the surface condition of egg shells in accordance with this embodiment is comprised of a rotating/conveying means 3 for conveying chicken eggs 2 serving as eggs in an X direction while rotating them; a radiating means 6 for radiating in a time-sharing manner a plurality of electromagnetic waves whose wavelengths are mutually different and which have substantially line spectra, i.e., two visible light including red light 4 and blue light 5 in this embodiment, to the chicken eggs 2 being conveyed by the rotating/conveying means 3; a detecting means 9 for detecting the type of stain or dirt 61 on egg shell surfaces and the degree of stain or dirt 61 on the egg shell surfaces as the surface condition of the egg shells of the chicken eggs 2 on the basis of the intensity of rays of reflected light 7 which are reflected electromagnetic waves reflected from the chicken eggs 2 due to the radiation of the red light 4 by the radiating means 6 as well as the intensity of rays of reflected light 8 which are reflected electromagnetic waves reflected from the chicken eggs 2 due to the radiation of the blue light 5 by the radiating means 6; an encoder 10 which is a conveyance detecting means for detecting the conveyance of the chicken eggs 2 by the rotating/conveying means 3; and an instructing/notifying means 11 which, on the basis of a detection signal from the encoder 10, instructs the radiating means 6 to effect the alternate (time-sharing) radiation of the red light 4 and the blue light 5 to the chicken eggs 2 and consecutively notifies the alternate ration of the red light 4 and the blue light 5 and the conveyance of the chicken eggs 2 by the rotating/conveying means 3.

The rotating/conveying means 3 includes a plurality of drum-shaped freely rolling members 12 arranged in the X direction and in a Y direction perpendicular to the X direction, as well as a rotating/traveling means 13 for causing the freely rolling members 12 to travel in the X direction and for rotating the freely rolling members 12. The rotating/traveling means 13 includes an endless chain 19 for holding the freely rolling members 12 rotatably; a pair of sprocket wheels 14 and 15 between which the endless chain 19 is trained; an electric motor 16 connected to a rotating shaft of the sprocket wheel 14 by means of pulleys, a belt, and the like; and a contact plate 18 which is in contact with the freely rolling members 12 traveling in an area A where the red light 4 and the blue light 5 are radiated from the radiating means 6 and which causes the freely rolling members 12 to rotate in a B direction as the endless chain 19 travels in the X direction. Between the freely rolling members 12 which are adjacent to each other in the X direction, the chicken eggs 2 are respectively adapted to be held with their longitudinal directions oriented in the Y direction.

The rotating/conveying means 3 is arranged such that as the electric motor 16 is operated, the sprocket wheel 14 is rotated by means of the pulleys, the belt, and the like, and as the sprocket wheel 14 is rotated, the endless chain 19 is caused to travel in the X direction, so as to convey the chicken eggs 2 placed on the freely rolling members 12 in the X direction. At the same time, the rotating/conveying means 3 is arranged such that as the endless chain 19 travels in the X direction, the freely rolling members 12 which consecutively come into contact with the contact plate 18 are caused to rotate in the B direction, thereby causing the chicken eggs 2 placed between the freely rolling members 12 which are adjacent to each other in the X direction to undergo at least a half rotation about their long axes in a C direction in the area A.

The encoder 10 is adapted to convert the rotation of the sprocket wheel 14 into an electrical signal and to supply the signal to the instructing/notifying means 11 as a detection signal.

The radiating means 6 includes a plurality of red-light emitting diodes 20 for emitting the red light 4; a pair of substrates 21 and 22 which are disposed above both sides of the endless chain 19 and on which the red-light emitting diodes 20 serving as red-light emitting elements are mounted; a plurality of blue-light emitting diodes 23 for emitting the blue light 5; and a pair of substrates 24 and 25 which are disposed above both sides of the endless chain 19 and on which the blue-light emitting diodes 23 serving as blue-light emitting elements are mounted. The substrates 21 and 22 are arranged such that their respective surfaces 27 which oppose each other in the Y direction and on which the red-light emitting diodes 20 are mounted are faced to the chicken eggs 2 on the freely rolling members 12 in the area A. The substrates 24 and 25 are similarly arranged such that their respective surfaces 28 which oppose each other in the Y direction and on which the blue-light emitting diodes 23 are mounted are faced to the chicken eggs 2 on the freely rolling members 12 in the predetermined area A. As a result, the radiating means 6 is adapted to radiate the emitted red light 4 and blue light 5 to the chicken eggs 2 independently of each other.

The instructing/notifying means 11 includes a signal generating means 31 which, in response to the detection signal from the encoder 10, generates drive signals for causing the red-light emitting diodes 20 and the blue-light emitting diodes 23 to emit light alternately (in a time-sharing manner) each time new chicken eggs 2 are conveyed to the area A, and which, in correspondence with the generation of the drive signals, transmits to the detecting means 9 a notification signal for notifying that light emission has been effected by the red-light emitting diodes 20 and the blue-light emitting diodes 23, and a pair of driving means 32 and 33 for causing the red-light emitting diodes 20 and the blue-light emitting diodes 23 to emit light in accordance with the drive signals from the signal generating means 31.

The radiating means 6 is adapted to cause the red-light emitting diodes 20 and the blue-light emitting diodes 23 to emit light by the driving means 32 and 33 which are driven in response to the detection signal from the encoder 10 through the instructing/notifying means 11, and to radiate the respective red light 4 and blue light 5 in the time-sharing manner as the chicken eggs 2 are conveyed and rotated by the rotating/conveying means 3.

The detecting means 9 has an imaging device 41 serving as a sensing and generating device which senses the intensity of the reflected light 7 from the chicken eggs 2, and generates light-and-dark image data on each of the relevant chicken eggs 2 based on the intensity of the reflected light 7 and which similarly senses the intensity of the reflected light 8 having wavelengths different from those of the reflected light 7, and generates other light-and-dark image data on each of the relevant chicken eggs 2 based on the intensity of the reflected light 8; an analyzing means 57 for analyzing the type of stain or dirt 61 on the egg shell surfaces of the chicken eggs 2 and the degree of the stain or dirt 61 on the egg shell surfaces on the basis of the two pieces of light-and-dark image data on each of the chicken eggs 2 based on the intensities of the reflected light 7 and 8 from the imaging device 41 and on the basis of the notification signal from the signal generating means 31 of the instructing/notifying means 11; and a display means 58 for displaying the type of stain or dirt 61 on the egg shell surfaces and the degree of the stain or dirt 61 on the egg shell surfaces as the results of analysis by the analyzing means 57.

The imaging device 41 has a monochrome camera (black-and-white camera) disposed above the area A so as to image the chicken eggs 2 in the area A, and is adapted to generate two pieces of light-and-dark image data on each of the chicken eggs 2 based on the intensities of the reflected light 7 and 8 and supply them to the analyzing means 57.

On the basis of the notification signal from the signal generating means 31, the analyzing means 57 stores for the respective chicken eggs 2 the light-and-dark image data based on the reflected light 7 from the imaging device 41 and the light-and-dark image data based on the reflected light 8, and analyzes the type of stain or dirt 61 on the egg shell surfaces of the respective chicken eggs 2 and the degree of the stain or dirt 61 on the egg shell surfaces on the basis of the stored light-and-dark image data based on the reflected light 7 and 8.

More specifically, the analyzing means 57 first stores the light-and-dark image data on one half of the egg shell surfaces of the relevant chicken eggs 2 generated by the reflected light 7 and 8 from the chicken eggs 2 on the basis of the red light 4 and the blue light 5 radiated upon arrival of the chicken eggs 2 in the area A. The analyzing means 57 then stores the light-and-dark image data on the remaining half of the egg shell surfaces of the relevant chicken eggs 2 generated by the reflected light 7 and 8 from the chicken eggs 2 on the basis of the red light 4 and the blue light 5 radiated when the chicken eggs 2 have undergone half rotation after having been conveyed in the X direction while being rotated. Thus the analyzing means 57 obtains two pieces of light-and-dark image data based on the intensities of the reflected light 7 and 8 with respect to the entire surfaces of the egg shell surfaces of the relevant chicken eggs 2, and determines the type of stain or dirt 61 on the egg shell surfaces of the relevant chicken eggs 2 on the basis of light and dark in the light-and-dark image data on the relevant chicken eggs 2 based on the intensity of the reflected light 7 and on the basis of light and dark in the light-and-dark image data on the relevant chicken eggs 2 based on the intensity of the reflected light 8. At the same time, the analyzing means 57 determines the degree of the stain or dirt 61 on the egg shell surfaces of the relevant chicken eggs 2 on the basis of a ratio of either one of light and dark in the light-and-dark image data on the relevant chicken eggs 2 based on the intensity of the reflected light 7, e.g., dark, and on the basis of a ratio of either one of light and dark in the light-and-dark image data on the relevant chicken eggs 2 based on the intensity of the reflected light 8, e.g., dark, and consecutively performs this determination with respect to the chicken eggs 2 which arrive in the area A.

For example, in a case where the stain or dirt 61 due to the yolk is present on the white egg shell surface of a white chicken egg (a chicken egg whose shell surface is white) 2 which arrived in the area A, the white color of the egg shell surface consists of all the color components, while the color of the yolk on the egg shell surface consists of color components of red and green (practically does not contain a blue color). Therefore, the light-and-dark image data based on the reflected light 7 consists of a light portion based on the intensity of the reflected light 7 in which practically all of the red light 4 has been reflected by the white portion of the egg shell surface and an intermediate portion between light and dark based on the intensity of the reflected light 7 in which part of the red light 4 has been reflected by the yolk portion on the egg shell surface. Meanwhile, the light-and-dark image data based on the reflected light 8 consists of a light portion based on the intensity of the reflected light 8 in which practically all of the blue light 5 has been reflected by the white portion of the egg shell surface and a dark portion based on the intensity of the reflected light 8 (in which case the reflected light is practically nil) in which the blue light 5 has been reflected by the yolk portion on the egg shell surface. Consequently, if the data is two kinds of such light-and-dark image data, the analyzing means 57 determines that the stain or dirt 61 on the egg shell surface is the stain or dirt 61 due to the yolk, and determines the degree of the stain or dirt 61 on the egg shell surface on the basis of the proportion of a dark (or intermediate portion) area in the light-and-dark image data with respect to the entire egg shell surface of the chicken egg 2.

Similarly, in a case where the stain or dirt 61 due to chicken droppings is present on the egg shell surface of the white chicken egg 2 which arrived in the area A, since the color of the chicken droppings on the egg shell surface consists of color components of a black color including gray color, the light-and-dark image data based on the reflected light 7 consists of a light portion based on the intensity of the reflected light 7 in which practically all of the red light 4 has been reflected by the white portion of the egg shell surface and a dark portion based on the intensity of the reflected light 7 (in which case the reflected light is practically nil) in which the red light 4 has been reflected by the portion of the chicken droppings on the egg shell surface. Meanwhile, the light-and-dark image data based on the reflected light 8 consists of a light portion based on the intensity of the reflected light 8 in which practically all of the blue light 5 has been reflected by the white portion of the egg shell surface and a dark portion based on the intensity of the reflected light 8 (in which case the reflected light is practically nil) in which the blue light 5 has been reflected by the portion of the chicken droppings on the egg shell surface. Consequently, if the data is two kinds of such light-and-dark image data, the analyzing means 57 determines that the stain or dirt 61 on the egg shell surface is the stain or dirt 61 due to the chicken droppings, and determines the degree of the stain or dirt 61 on the egg shell surface on the basis of the proportion of a dark area in the light-and-dark image data with respect to the entire egg shell surface of the chicken egg 2.

In addition, in a case where the data concerning the white chicken egg 2 are two kinds of light-and-dark image data other than those described above, the analyzing means 57 determines that the stain or dirt 61 on the egg shell surface is other type of stain or dirt 61, and determines the degree of the other type of stain or dirt 61 on the egg shell surface in the same way as described above. Thus the analyzing means 57 is adapted to detect the presence or absence, type, and degree of the stain or dirt 61 on the egg shell surface of each of the chicken eggs 2 which are conveyed in the X direction.

In addition, on the basis of at least one of a reflected area in which the red light has been totally reflected in the light-and-dark image data based on the reflected light 7 and a reflected area in which the blue light has been totally reflected in the light-and-dark image data based on the reflected light 8 per white chicken egg 2, the analyzing means 57 may determine the shape, size, and the like of the relevant chicken egg 2. In addition, on the basis of the reflected areas of the chicken egg 2 with respect to the red light 4 and the blue light 5 and the intensities of reflection in the areas, the analyzing means 57 may determine that the stain or dirt 61 is absent in the portion where the red light 4 and the blue light 5 are reflected, that black or gray stain or dirt 61 due mainly to chicken droppings adheres to the portion where the red light 4 and the blue light 5 is not reflected, that yellow stain or dirt 61 due mainly to such as the yoke of the chicken egg 2 is present in the portion where the red light 4 is reflected but the blue light 5 is not reflected, and that an unexpected foreign matter other than those mentioned above adheres to the portion where the blue light 5 is reflected but the red light 4 is not reflected.

The results of analysis by the analyzing means 57 are transmitted to the display means 58, and the display means 58 displays the results of analysis. It should be noted that in the case where the apparatus 1 for detecting the surface condition in accordance with this embodiment is incorporated in the washing, sorting, and packaging processes, preferably in the case where it is incorporated prior to the washing process, if the apparatus 1 for detecting the surface condition has detected the chicken egg 2 having the stain or dirt 61 due to the yolk on the egg shell surface and/or the chicken egg 2 having the stain or dirt 61 due to the chicken droppings of a fixed size or more on the egg shell surface, such a chicken egg 2 may be automatically rejected without moving it to the washing process.

In accordance with the above-described apparatus 1 for detecting the surface condition, since the surface condition of the egg shells is detected on the basis of the intensities of the reflected light 7 and 8 after radiating the red light 4 and the blue light 5 to the chicken eggs 2, no mechanical shocks are imparted to the chicken eggs 2, and no new cracks are caused in the egg shells in performing the detection. Moreover, since the surface condition of the egg shells is detected on the basis of the intensities of the reflected light 7 and 8 based on the red light 4 and the blue light 5 having different wavelengths, it is possible to discriminate and detect accurately and with extremely high reliability the surface condition of the egg shells concerning the stain or dirt 61 due to the yoke, chicken droppings, and other foreign matter adhering to the egg shell surfaces.

It should be noted that, in the apparatus 1 for detecting the surface condition in accordance with the embodiment, the radiating means 6 may be adapted to separately irradiate the chicken eggs 2 with infrared rays, far infrared rays, ultraviolet rays, visible rays, and other electromagnetic waves having various wavelengths. In addition, the imaging device 41 may be embodied by using a color camera instead of the black-and-white camera. In this case, the detecting means 9 may be provided with analyzing conditions so as to analyze differences in the hue, saturation, lightness, and the like in the image data based on the reflected light from the egg shells of the chicken eggs 2.

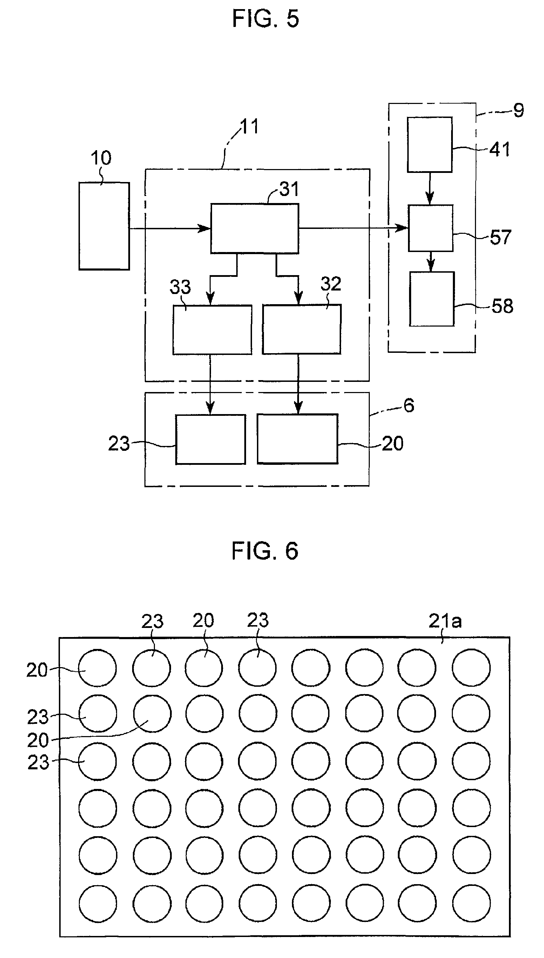

Further, the radiating means 6 may be provided with a substrate 21a, such as the one shown in Fig. 6, on which the plurality of red-light emitting diodes 20 and the plurality of blue-light emitting diodes 23 are alternately arranged and mounted, instead of the substrates 21, 22, 24, and 25. In addition, at the same time as the transmission of the drive signals to the driving means 32 and 33 by the signal generating means 31, signals for instructing the starting and stopping of imaging may be transmitted from the signal generating means 31 to the imaging device 41 so as to start the imaging of the chicken eggs 2 by the imaging device 41 at the same time as the starting of the emission of the red light 4 by the red-light emitting diodes 20 and to stop the imaging of the chicken eggs 2 by the imaging device 41 at the same time as the completion of the emission of the blue light 5 by the blue-light emitting diodes 23. In addition, although a description has been given of the example of the apparatus for detecting the surface condition of the white chicken eggs 2 whose egg shell surfaces are white, the stain or dirt 61 and the like on the egg shell surfaces of, for instance, brown eggs may be detected by such as changing appropriately the wavelengths of the electromagnetic waves radiated by the radiating means 6 and the analyzing conditions of the analyzing means 57.

## Claims

1. A method of detecting the surface condition of an egg shell, comprising the steps of:
radiating to an egg a plurality of electromagnetic waves whose wavelengths are mutually different and each of which has substantially line spectrum; and
detecting the surface condition of the egg shell on the basis of an intensity of a reflected electromagnetic wave from the egg due to the radiation.

2. The method of detecting the surface condition of an egg shell according to claim 1, wherein the radiating step includes the steps of radiating the plurality of electromagnetic waves to the egg in a time-sharing manner.

3. The method of detecting the surface condition of an egg shell according to claim 1, wherein the radiating step includes the steps of radiating the plurality of electromagnetic waves to the egg simultaneously.

4. The method of detecting the surface condition of an egg shell according to any one of claims 1 to 3, wherein the radiating step includes the steps of radiating the plurality of electromagnetic waves to the egg independently of each other.

5. The method of detecting the surface condition of an egg shell according to any one of claims 1 to 4, wherein the electromagnetic waves include light, in particular visible light, preferably selected from red light, blue light, and green light, most preferably red light and blue light.

6. The method of detecting the surface condition of an egg shell according to any one of claims 1 to 5, wherein the surface condition of the egg shell to be detected includes one or more of the presence or absence of stain or dirt on an egg shell surface, a type of stain or dirt on an egg shell surface and a degree of stain or dirt on an egg shell surface.

7. The method of detecting the surface condition of an egg shell according to any one of claims 1 to 6, further comprising the steps of rotating the egg, and said radiating step including the steps of radiating the electromagnetic waves to the rotated egg in correspondence with the rotation.

8. The method of detecting the surface condition of an egg shell according to any one of claims 1 to 7, further comprising the steps of conveying the egg, and the radiating step including the steps of radiating the electromagnetic waves to the conveyed egg in correspondence with the conveyance.

9. The method of detecting the surface condition of an egg shell according to any one of claims 1 to 8, wherein the detecting step includes the steps of generating image data on the egg on the basis of the intensity of the reflected electromagnetic wave, and detecting the surface condition of the egg shell from the image data.

10. The method of detecting the surface condition of an egg shell according to claim 9, wherein the image data on the egg includes light-and-dark image data.

11. The method of detecting the surface condition of an egg shell according to claim 10, wherein the detecting step includes the steps of detecting the surface condition of the egg shell on the basis of one piece of light-and-dark image data on the egg based on the intensity of one reflected electromagnetic wave and another piece of light-and-dark image data on the egg based on the intensity of another reflected electromagnetic wave having a wavelength different from that of the one reflected electromagnetic wave.

12. The method of detecting the surface condition of an egg shell according to claim 11, wherein the detecting step includes the steps of detecting the surface condition of the egg shell on the basis of light and dark in the one piece of light-and-dark image data and light and dark in the other piece of light-and-dark image data.

13. The method of detecting the surface condition of an egg shell according to claim 11 or 12, wherein the detecting step includes the steps of detecting the surface condition of the egg shell on the basis of at least one of a proportion of a light area and a proportion of a dark area in at least one of the one piece of light-and-dark image data and the other piece of light-and-dark image data.

14. An apparatus for detecting the surface condition of an egg shell, comprising:
radiating means for radiating to an egg a plurality of electromagnetic waves whose wavelengths are mutually different and each of which has substantially line spectrum; and
detecting means for detecting the surface condition of the egg shell on the basis of an intensity of a reflected electromagnetic wave reflected from the egg due to the radiation by said radiating means.

15. The apparatus for detecting the surface condition of an egg shell according to claim 14, wherein said radiating means radiates the plurality of electromagnetic waves to the egg in a time-sharing manner.

16. The apparatus for detecting the surface condition of an egg shell according to claim 14, wherein said radiating means radiates the plurality of electromagnetic waves to the egg simultaneously.

17. The apparatus for detecting the surface condition of an egg shell according to any one of claims 14 to 16, wherein said radiating means radiates the plurality of electromagnetic waves to the egg independently of each other.

18. The apparatus for detecting the surface condition of an egg shell according to any one of claims 14 to 17, wherein said radiating means radiates light as the electromagnetic wave, in particular visible light, preferably red light, blue light, and green light, most preferably red light and blue light.

19. The apparatus for detecting the surface condition of an egg shell according to claim 18, wherein said radiating means has a plurality of light emitting elements disposed in such a manner as to radiate the emitted light to the egg, said elements preferably being a red-light emitting element for emitting red light and a blue-light emitting element for emitting blue light, and preferably arranged with a substrate on which a plurality of red-light emitting elements and a plurality of blue-light emitting elements are alternately arranged and mounted.

20. The apparatus for detecting the surface condition of an egg shell according to any one of claims 14 to 19, wherein said detecting means detects one or more of the presence or absence of stain or dirt on an egg shell surface as the surface condition of the egg shell, a type of stain or dirt on an egg shell surface as the surface condition of the egg shell, or a degree of stain or dirt on an egg shell surface as the surface condition of the egg shell.

21. The apparatus for detecting the surface condition of an egg shell according to any one of claims 14 to 20, further comprising: rotating means for rotating the egg, wherein said radiating means radiates the electromagnetic waves in correspondence with the rotation of the egg by said rotating means.

22. The apparatus for detecting the surface condition of an egg shell according to any one of claims 14 to 21, further comprising: conveying means for conveying the egg, wherein said radiating means radiates the electromagnetic waves in correspondence with the conveyance of the egg by said conveying means.

23. The apparatus for detecting the surface condition of an egg shell according to claim 22, further comprising:
conveyance detecting means for detecting the conveyance of the egg by said conveying means, wherein said radiating means radiates the electromagnetic waves in response to a detection signal from said conveyance detecting means, and said detecting means detects the surface condition of the egg shell in response to the detection signal from said conveyance detecting means.

24. The apparatus for detecting the surface condition of an egg shell according to claim 22 or 23, wherein said conveying means has a plurality of freely rolling members arranged in a conveying direction of the egg and a direction perpendicular to the conveying direction and traveling means for causing said freely rolling members to travel, the egg being held between said freely rolling members which are adjacent to each other in the conveying direction.

25. The apparatus for detecting the surface condition of an egg shell according to any one of claims 14 to 24, wherein said detecting means has a sensing and generating device for sensing the intensity of the reflected electromagnetic waves from the egg and generating image data on the egg, and analyzing means for analyzing the surface condition of the egg shell on the basis of the image data from said sensing and generating device.

26. The apparatus for detecting the surface condition of an egg shell according to claim 25, wherein said sensing and generating device generates light-and-dark image data as the image data on the egg.

27. The apparatus for detecting the surface condition of an egg shell according to claim 26, wherein said sensing and generating device generates one piece of light-and-dark image data on the egg based on the intensity of one reflected electromagnetic wave and another piece of light-and-dark image data on the egg based on the intensity of another reflected electromagnetic wave having a wavelength different from that of the one reflected electromagnetic wave, and said analyzing means analyzes the surface condition of the egg shell on the basis of the two pieces of light-and-dark image data.

28. The apparatus for detecting the surface condition of an egg shell according to claim 27, wherein said analyzing means determines the surface condition of the egg shell on the basis of light and dark in the one piece of light-and-dark image data and light and dark in the other piece of light-and-dark image data.

29. The apparatus for detecting the surface condition of an egg shell according to claim 27 or 28, wherein said analyzing means determines the surface condition of the egg shell on the basis of at least one of a proportion of a light area and a proportion of a dark area in at least one of the one piece of light-and-dark image data and the other piece of light-and-dark image data.
